(19) Europäisches Patentamt
European Patent Office
Office européen des brevets

(11) Publication number : **0 465 189 A2**

# (12) EUROPEAN PATENT APPLICATION

(21) Application number : **91305939.0**

(22) Date of filing : **01.07.91**

(51) Int. Cl.$^5$ : **C12N 15/52,** C12N 9/00, C07K 15/00, C12P 21/02

(30) Priority : **06.07.90 US 549502**

(43) Date of publication of application : **08.01.92 Bulletin 92/02**

(84) Designated Contracting States : **AT BE CH DE DK ES FR GB GR IT LI NL SE**

(71) Applicant : **ELI LILLY AND COMPANY**
**Lilly Corporate Center**
**Indianapolis Indiana 46285 (US)**

(72) Inventor : **Kovacevic, Steven**
**5620 Broadway**
**Indianapolis, Indiana 46220 (US)**
Inventor : **Miller, James Robert**
**4228 Glenview Drive, South**
**Indianapolis, Indiana 46250 (US)**

(74) Representative : **Hudson, Christopher Mark et al**
**Erl Wood Manor**
**Windlesham Surrey GU20 6PH (GB)**

## (54) Recombinant DNA expression vectors and DNA compounds that encode deacetylcephalosporin C synthase activity.

(57) The present invention provides DNA compounds that encode hydroxylase activity of Streptomyces clavuligerus. The compounds can be used to construct recombinant DNA expression vectors for a wide variety of host cells, including E. coli, Penicillium, Streptomyces, Aspergillus, and Cephalosporium.

EP 0 465 189 A2

The natural sulfur-containing β-lactam antibiotics are the most important clinically, and the isolation of novel β-lactam compounds continues six decades after the discovery of the penicillins by Fleming. The common structural feature of the penicillins and cephalosporins (including cephamycins) is the β-lactam ring.

These antibiotics are produced by a variety of prokaryotes and lower eukaryotes. The penicillins, exemplified by the compounds penicillin G or penicillin V, are produced by filamentous fungi, most notably Penicillium chrysogenum. The cephalosporins, first isolated as a product from the lower eukaryote, Cephalosporium acremonium (syn. Acremonium chrysogenum), are also metabolites of many prokaryotes but especially Streptomyces clavuligerus, S. lipmanii and S. cattleya that also produce cephamycins and other β-lactams such as oxypenams (clavulanic acid) and carbapenems (thienamycin).

The development of cell-free systems from β-lactam-producing organisms has allowed the establishment of the biosynthetic steps in the pathway of the sulfur-containing β-lactams (penicillins and cephalosporins, including cephamycins).

The initial steps in the formation of penicillins in filamentous fungi (e.g. P. chrysogenum), and the cephalosporins (e.g., S. clavuligerus) produced by both prokaryotes and lower eukaryotes (e.g., C. acremonium), are identical. ACV synthetase catalyzes the condensation of the amino acid precursors L-α-aminoadipate, L-cysteine, and L-valine to the tripeptide LLD-ACV. The next step forms the first β-lactam in the pathway by the cyclization of the tripeptide yielding isopenicillin N (IPN), a precursor to all penicillins, cephalosporins and cephamycins.

After synthesis of IPN, the pathways to cephalosporins and penicillins diverge. In Penicillium chrysogenum, for example, the α-aminoadipyl side chain of IPN can be exchanged for one of many (nearly 100 to date) hydrophobic side chains derived from the corresponding acyl CoA. One of the most familiar examples is,the formation of penicillin G from phenylacetyl CoA and IPN. However, in the fungus C. acremonium, the α-aminoadipyl side chain is isomerized to produce penicillin N. The five-membered thiazolidine ring of the penicillin is then "expanded" to the six-membered dihydrothiazine ring that is characteristic of the cephalosporins. This reaction is catalyzed by deacetoxycephalosporin C synthetase (DAOCS) and produces the first cephalosporin in the pathway, deacetoxycephalosporin C (DAOC). For chemical structures in the pathway see Figure 1.

The present invention provides DNA compounds comprising isolated DNA sequences encoding an activity involved in the production of cephalosporins, deacetylcephalosporin C synthase (hydroxylase). The present invention expands the repertoire of beta-lactam biosynthetic enzymes which can be overproduced. This ability facilitates the bioconversion of substrate analogs to novel beta-lactams and strain improvement by increased gene dosage.

The present invention provides DNA compounds comprising isolated DNA sequences that encode hydroxylase activity from Streptomyces clavuligerus. Hydroxylase catalyzes the reaction in which DAOC is hydroxylated at the 3-methyl group to form deacetylcephalosporin C (DAC). The reaction catalyzed is a critical step in the biosynthesis of important antibiotics, such as cephalosporins from Cephalosporium acremonium and 7α-methoxycephalosporins or cephamycins from Streptomyces clavuligerus and S. lipmanii. Thus, it is quite important to be able to produce the activity in large quantities.

The DNA compounds of the present invention encode the hydroxylase activity in a single open reading frame. Transcription of this open reading frame, followed by translation of the resulting mRNA, yields a single polypeptide chain that possesses hydroxylase activity. A preferred DNA compound of the invention that encodes the hydroxylase activity was isolated from Streptomyces clavuligerus genomic DNA and used to construct a recombinant DNA expression vector called pOW399. Plasmid pOW399 is part of the present invention. The cloned hydroxylase gene is useful for increasing the yield of cephalosporins in fungi and bacteria, particularly if the reaction catalyzed by hydroxylase is a rate-limiting step.

The E. coli-produced hydroxylase activity likewise catalyzes the formation of DAC from DAOC. Crude cell extracts of the E. coli transformants of the invention exhibit hydroxylase activity. These E. coli expression vectors and transformants provide an efficient means for obtaining large amounts of active hydroxylase. The hydroxylase is useful not only for the production of DAC but also for the increased yield in production of other compounds of the beta-lactam antibiotic pathway.

The DNA compound encoding the hydroxylase activity is readily modified to construct expression vectors that increase the yield of hydroxylase in a variety of other organisms, including, for example, Escherichia, Penicillium, Cephalosporium, Paecilomyces and Streptomyces, especially S. clavuligerus. The construction protocols utilized for the E. coli vector of the invention can be followed to construct analogous vectors for other organisms, merely by substituting, if necessary, the appropriate regulatory elements using techniques well-known to skilled artisans.

The present invention also provides a method for constructing a recombinant host cell capable of expressing hydroxylase activity of Streptomyces clavuligerus, said method comprising transforming a host cell with a recombinant DNA vector that comprises an isolated DNA sequence encoding hydroxylase activity of Streptomyces clavuligerus. Preferred host cells include E. coli, Cephalosporium, Streptomyces, Aspergillus and Penicillium. Such transformed host cells may be cultured under conditions well-known to skilled artisans such that the hydroxylase gene is expressed, thus producing hydroxylase activity in the recombinant host cell.

Therefore, also provided by the present invention is a method for expressing hydroxylase activity of Streptomyces clavuligerus in a recombinant host cell, said method comprising culturing said transformed host cell under conditions suitable for gene expression.

The following section provides a more detailed description of the present invention. For purposes of clarity and as an aid in the understanding of the invention, as disclosed and claimed herein, the following items are defined below.

AmdS - an acetamidase gene.

cl857 - a gene encoding a temperature sensitive repressor of the λpL promoter.

DAC - deacetylcephalosporin C, the structure of which is depicted below:

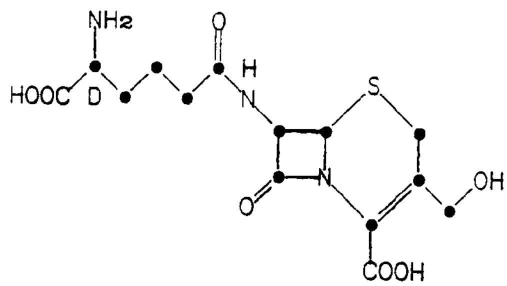

DACS or DACS Activity - deacetylcephalosporin C synthase, the enzymatic activity that catalyzes conversion of DAOC to DAC, also called hydroxylase.

DAOC - deacetoxycephalosporin C, the structure of which is depicted below:

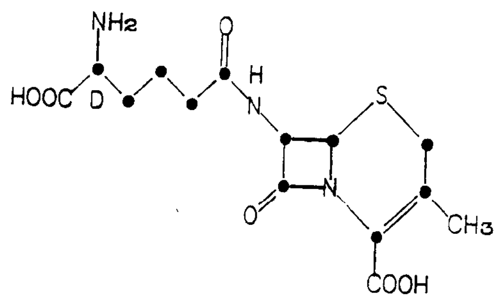

Hydroxylase or Hydroxylase Activity - the enzymatic activity that catalyzes conversion of DAOC to DAC; also called DACS.

ORI - a plasmid or vector origin of replication, the DNA sequence that serves as an attachment or start site for DNA polymerase.

pL - the leftward promoter from bacteriophage lambda.

Isolated DNA Sequence - Any DNA sequence, however constructed or synthesized, which is locationally distinct from its natural location in genomic DNA. The definition includes the isolated DNA sequence in all its forms other than the natural state. For example, the DNA sequence may be inserted into a plasmid or phage vector or inserted into the genome of the organism from which it came or any other organism to increase the gene dosage.

TcR - the tetracycline resistance-conferring gene.

The restriction site and function maps presented in the accompanying drawings is an approximate representation of the recombinant DNA vectors discussed herein. The restriction site information is not exhaustive; therefore, there may be more restriction sites of a given type on the vector than actually shown on the map.

Figure 1. Beta-lactam biosynthesis.

Figure 2. A restriction site and function map of plasmid pOW399.

The present invention provides DNA compounds and recombinant DNA cloning and expression vectors that comprise isolated DNA sequences encoding the hydroxylase activity of Streptomyces clavuligerus. The sequence of the S. clavuligerus hydroxylase-encoding DNA is depicted below. In the depiction, only the "sense"

or coding strand of the double-stranded DNA molecule is shown, and the DNA is depicted from left to right in the 5′ → 3′ orientation. The nucleotide sequence is numbered; the numbers appear to the left of the DNA sequence. The amino acid residue sequence of hydroxylase encoded by the DNA is listed, following the DNA sequence, from left to right in the amino-terminus → carboxyl-terminus direction.

| | | | | | |
|---|---|---|---|---|---|
| 1 | ATGGCGGACA | CGCCCGTACC | GATCTTCAAC | CTCGCCGCAC | TGCGGGAAGG |
| 51 | CGCCGATCAG | GAGAAGTTCC | GCGAGTGCGT | GACCGGGATG | GGGGTCTTCT |
| 101 | ACCTCACCGG | GTACGGCGCC | GGGGATAAGG | ACCACCGGCT | GGCCACGGAC |
| 151 | ACGGCGATGG | ACTTCTTCGC | GAACGGCACC | GAGGCCGAGA | AGGCGGCCGT |
| 201 | GACCACGGAC | GTCCCGACCA | TGCGGCGCGG | CTACTCCGCG | CTGGAGGCCG |
| 251 | AGAGCACCGC | CCAGGTGACC | AGGACCGGTT | CCTACACGGA | CTACTCGATG |
| 301 | TCCTTCCCAT | GGGCATCTCG | GGCAACGTCT | TCCCCTCGCC | GGAGTTCGAG |
| 351 | CGGGTGTGGA | CGGAGTACTT | CGACAAGCTC | TACGCGGCGG | CCCAGGAGAC |
| 401 | GGCGCGGCTG | GTGCTGACCG | CGAGCGGCGG | CTATGACGCG | GAGATCGTCG |
| 451 | GAAGCCTGGA | CGAGCTGCTG | GACGCCGACC | CCGTGCTGCG | GCTGCGGTAC |
| 501 | TTCCCCGAGG | TGCCCGAGCA | CCGGTCCGCC | GAGCACGAGC | CGCGCCGGAT |
| 551 | GGCCCCGCAC | TACGACCTGT | CGATCATCAC | CTTCATCCAC | CAGACGCCGT |
| 601 | GCGCCAACGG | CTTCGTCAGC | CTCCAGGCCG | AGATCGGCGG | CGAACTGGTG |
| 651 | AGCCTGCCCG | TCGTGGAGGA | CGCCGTCGTC | GTGATGTGCG | GCGCGATGGC |
| 701 | CCCGCTGGCG | ACCCAGGGCG | CGCTGCCCGC | GCCCCGGCAC | CACGTCCGGT |
| 751 | CCCCCGGCGC | CGGTATGCGC | GAGGGCAGCG | ACCGCACGTC | GAGCGTCTTC |
| 801 | TTCCTGCGCC | CCACGACCGA | CTTCTCGTTC | TCGGTGGCCA | AGGCCCGCAG |
| 851 | CTACGGCCTC | GCCGTCGACC | TCGACATGGA | GACGGCCACC | TTCGGCGACT |
| 901 | GGATCGGCAC | CAACTACGTC | ACCATGCACG | CGAAGAACGA | GCCGCAGGCC |
| 951 | GGATGA | | | | |

wherein A is a deoxyadenyl residue, G is a deoxyguanyl residue, C is a deoxycytidyl residue, and T is a thymidyl residue.

Following is the amino acid sequence encoded by the preceding DNA sequence:

H-MetAlaAspThrProValProIlePheAsnLeuAlaAlaLeuArgGluGlyAlaAspGln
GluLysPheArgGluCysValThrGlyMetGlyValPheTyrLeuThrGlyTyrGlyAla
GlyAspLysAspHisArgLeuAlaThrAspThrAlaMetAspPhePheAlaAsnGlyThr
GluAlaGluLysAlaAlaValThrThrAspValProThrMetArgArgGlyTyrSerAla
LeuGluAlaGluSerThrAlaGlnValThrArgThrGlySerTyrThrAspTyrSerMet
SerPheSerMetGlyIleSerGlyAsnValPheProSerProGluPheGluArgValTrp
ThrGluTyrPheAspLysLeuTyrAlaAlaAlaGlnGluThrAlaArgLeuValLeuThr
AlaSerGlyGlyTyrAspAlaGluIleValGlySerLeuAspGluLeuLeuAspAlaAsp
AlaSerGlyGlyTyrAspAlaGluIleValGlySerLeuAspGluLeuLeuAspAlaAsp
ProValLeuArgLeuArgTyrPheProGluValProGluHisArgSerAlaGluHisGlu
ProArgArgMetAlaProHisTyrAspLeuSerIleIleThrPheIleHisGlnThrPro
CysAlaAsnGlyPheValSerLeuGlnAlaGluIleGlyGlyGluLeuValSerLeuPro
ValValGluAspAlaValValValMetCysGlyAlaMetAlaProLeuAlaThrGlnGly
AlaLeuProAlaProArgHisHisValArgSerProGlyAlaGlyMetArgGluGlySer
AspArgThrSerSerValPhePheLeuArgProThrThrAspPheSerPheSerValAla
LysAlaArgSerTyrGlyLeuAlaValAspLeuAspMetGluThrAlaThrPheGlyAsp
TrpIleGlyThrAsnTyrValThrMetHisAlaLysAsnGluProGlnAlaGly-OH

wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue.

Those skilled in the art will recognize that the DNA sequence depicted above is an important part of the present invention. The above sequence can be conventionally synthesized by the modified phosphotriester method using fully protected deoxyribonucleotide building blocks. Such synthetic methods are well known in the art and can be carried out in substantial accordance with the procedure of Itakura et al., 1977, Science 198:1056 and Crea et al., 1978, Proc. Nat'l Acad. Sci. USA 75:5765. In addition, an especially preferred method is disclosed in Hsiung et al., 1983, Nuc. Acid Res. 11:3227 and Narang et al., 1980, Methods in Enzymology 68:90. In addition to the manual procedures referenced above, the DNA sequence can be synthesized using automated DNA synthesizers, such as the ABS (Applied Biosystems, 850 Lincoln Centre Drive, Foster City, CA 94404) 380B DNA Synthesizers.

Due to the degenerate nature of the genetic code, which results from there being more than one codon for most of the amino acid residues and translation stop signal, the amino acid residue sequence of the hydroxylase enzyme depicted above can be encoded by a multitude of different DNA sequences. Because these alternate DNA sequences would encode the same amino acid residue sequence of the present invention, the present invention further comprises these alternate sequences.

A preferred hydroxylase activity-encoding DNA compound was isolated from a strain of Streptomyces clavuligerus. A genomic library of the total genomic DNA of the S. clavuligerus strain was constructed and examined for the presence of sequences homologous to a deoxyribooligonucleotide probe. This probe was constructed in accordance with information obtained about the amino-terminal amino acid sequence of the S. clavuligerus hydroxylase, with kowledge of the genetic code, and with kowledge of codon usage preferences of Streptomyces. DNA sequencing revealed which clone encoded the S. clavuligerus hydroxylase.

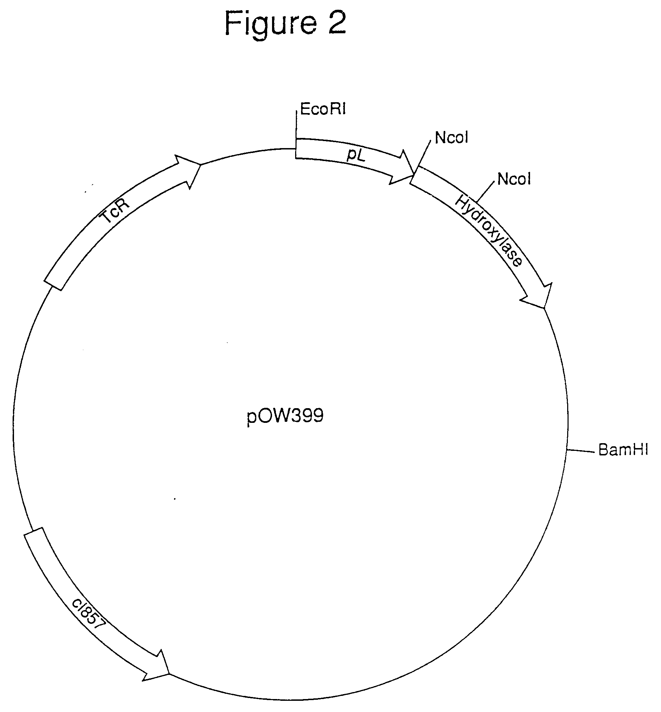

E. coli K12 JM109/pOW399, which contains the plasmid with the hydroxylase gene under the control of the λpL promoter, was deposited and made part of the stock culture collection of the Northern Regional Research Laboratories (NRRL), Peoria, IL 61604, on June 4, 1990, under the accession number NRRL B-18658. Plasmid pOW399 can be isolated from E. coli K12 JM109 by procedures well-kown by those skilled in the art. Plasmid pOW399 contains the intact Streptomyces clavuligerus hydroxylase gene, which can be isolated, for example, from the plasmid on an ~2.5 kb EcoRI/BamHI restriction fragment. A restriction site and function map of plasmid pOW399 is presented in Figure 2 of the accompanying drawings.

At low temperatures of about 30°C, the cI857 protein encoded on plasmid pOW399 or its derivatives is active and able to repress activity of the λpL promoter, but when the temperature is raised to about 42°C, the cI857 protein is inactivated, and the λpL promoter drives transcription of large amounts of mRNA encoding the gene product of interest. At temperatures of about 42°C, E. coli K12 JM109/pOW399 expresses hydroxylase activity at high levels, approaching ~5% of the total cell protein. Crude cell extracts from these E. coli K12 JM109/pOW399 transformants are able to catalyze the conversion of DAOC into DAC, whereas cell extracts from non-transformed E. coli K12 JM109 cells cannot catalyze this conversion. The method of assay and results of the assay for the conversion reaction are presented in Example 1.

Plasmid pOW399 is especially preferred for driving expression of hydroxylase activity in E. coli not only because of the high expression levels achieved when using the plasmid but also because of the selectable marker present on the plasmid. Many recombinant DNA vectors encode a β-lactamase, so that cells containing the vector can grow in the presence of certain β-lactam antibiotics, such as ampicillin. However, if one desires to use a cell-free extract containing hydroxylase activity for purposes of constructing β-lactams, one does not want the extract to contain β-lactamase activity. Thus, plasmid pOW399 does not encode a β-lactamase for a selectable marker but rather employs a tetracycline resistance-conferring gene, which encodes a protein that does not react with β-lactams.

E. coli is an efficient organism for producing large amounts of hydroxylase activity. Because culturing E. coli is less complex than culturing organisms that naturally produce hydroxylase, E. coli K12 JM109/pOW399 can be used to produce recombinant hydroxylase more efficiently and economically than non-recombinant or "natural" hydroxylase producers. This hydroxylase activity can be used for in vitro experiments to modify DAOC to form novel antibiotics or antibiotic core structures.

However, the present invention is not limited to the particular vector exemplified herein. Instead, the present invention comprises DNA compounds that encode the hydroxylase activity of Streptomyces clavuligerus. The DNA compounds of the present invention can be used to construct expression vectors that drive expression of hydroxylase activity in any host cell in which the expression vector replicates or integrates and in which the promoter and translational activating sequence are functional.

The present invention can therefore be used to increase the natural amounts of hydroxylase activity in the cell by increasing the number of genes which code for the hydroxylase enzyme. As a result, more DAOC can be converted to DAC. An increase in DAC could ultimately result in an increased yield of cephalosporin C and cephamycin C in the cell. Cephalosporin C and cephamycin are both antibiotics with known utility. This invention could also facilitate production of other antibiotics in which the hydroxylase enzyme or derivatives thereof function to catalyze intermediate steps in biosynthesis.

Another use of the present invention is to halt biosynthesis at DAOC so that DAOC can be used to generate new and useful cephalosporins. The biosynthetic pathway can be halted by disrupting or displacing the functional hydroxylase gene. Unstable vectors containing a mutated hydroxylase gene may undergo single or double cross-over events with the genomic copy of the hydroxylase gene when introduced into the cell. If a selectable marker is integrated into the hydroxylase coding sequence, some cells which retain the ability to grow under selective conditions will have had the hydroxylase gene disrupted.

The DNA compounds of the present invention can also be used as a probe to find hydroxylase, expandase (European Patent Publication No. 0341892, published November 15, 1989) or expandase/hydroxylase (European Patent Publication No. 0281391, published September 7, 1988) genes in other microorganisms.

The hydroxylase expression vectors of the present invention are not limited to a tetracycline resistance gene as the selectable marker. Those skilled in the art recognize that many selectable markers are suitable for use on hydroxylase expression vectors. Such selectable markers include, for example, genes that confer kanamycin or chloramphenicol resistance, or other antibiotic resistance-conferring genes.

The present invention comprises any E. coli expression plasmid or vector that drives expression of hydroxylase activity in E. coli. Thus, the present invention comprises expression vectors that drive expression of hydroxylase activity and utilize a replicon functional in E. coli, such as, for example, a replicon from such plasmids as pBR322 or the pUC series of plasmids. Nor is the present invention solely limited to plasmid vectors, because the present invention also comprises expression vectors that express hydroxylase activity and utilize integration or viral replication to provide for replication and maintenance in the host cell.

The present invention is not limited to a particular promoter and translational activating sequence to drive expression of the hydroxylase activity-encoding DNA. The present invention comprises the use of any promoter and translational activating sequence that function in E. coli and are used to express hydroxylase activity in E. coli. Many promoter and translational activating sequences functional in E. coli are known and are suitable for driving expression of hydroxylase activity in E. coli. Such transcriptional and translational activating sequences include, but are not limited to, the lpp, lac, trp, tac, λpL, and λpR promoter and translational activating sequences. Likewise, examples of suitable promoter sequences functional in other organisms are the promoter of

the IPNS gene from Penicillium chrysogenum and the promoter from the amdS gene in Aspergillus nidulans both of which are disclosed in U.S. Patent 4,892,819, herein incorporated by reference. Also useful is the promoter of the IPNS gene in Cephalosporium aeremonium, disclosed in U.S. Patent 4,885,251, herein incorporated by reference.

In addition, transcriptional and translational activating sequences from other organisms can be ligated to the present hydroxylase activity-encoding DNA compounds to form expression vectors that drive expression of hydroxylase activity in host cells in which the activating sequence functions. Although E. coli is the host best suited for hydroxylase production and subsequent purification for in vitro use, vectors that drive expression of hydroxylase activity in host cells other than E. coli are also useful, especially for purposes of increasing the antibiotic-producing ability and efficiency of a given organism.

A variety of organisms produce β-lactam antibiotics. The following Table presents a non-comprehensive list of β-lactam antibiotic-producing organisms.

TABLE I

β-Lactam Antibiotic-Producing Organisms

| Organism | Antibiotic |
|---|---|
| Agrobacterium | various β-lactams |
| Arachnomyces minimus | penicillins and cephalosporins |
| Anixiopsis peruviana | penicillins and cephalosporins |
| Cephalosporium acremonium, purpurascens, polyaleurum, chrysogenum, curtipes | penicillins and cephalosporins |
| Chromobacterium | various β-lactams |
| Emericellopsis terricola, minima, synnematicola, glabra, mirabilis, salmosynnemata | penicillins and cephalosporins |
| Flavobacterium | various β-lactams |
| Gluconobacter | various β-lactams |

Table I continued

| | |
|---|---|
| Nocardia | |
| lactamadurans | cephamycin C |
| uniformis | nocardicin |
| Paecilomyces | penicillins and cephalosporins |
| carneus | |
| persicinus | |
| Penicillium | |
| chrysogenum | various penicillins and other β-lactams |
| Serratia | various β-lactams |
| Spiroidium | penicillins and cephalosporins |
| fuscum | |
| Streptomyces | |
| antibioticus | clavulanic acid |
| argenteolus | asparenomycin A, MM 4550, and MM 13902 |
| cattleya | thienamycin |
| chartreusis | SF 1623 and cephamycin A and B |
| cinnamonensis | cephamycin A and B |
| clavuligerus | PA-32413-I, cephamycin C, A16886A, penicillins, cephalosporins, clavulanic acid, and other clavams |
| fimbriatus | cephamycin A and B |
| flavovirens | MM 4550 and MM 13902 |
| flavus | MM 4550 and MM 13902 |
| fulvoviridis | MM 4550 and MM 13902 |
| griseus | cephamycin A and B and carpetimycin A and B |
| halstedi | cephamycin A and B |
| heteromorphus | C2081X and cephamycin A and B |
| hygroscopicus | deacetoxy-cephalosporin C |
| lipmanii | cephamycin, penicillin N, 7-methoxycephalosporin C, A16884, MM4550, MM13902 |
| olivaceus | epithienamycin F, MM 4550, and MM 13902 |

Table I continued

| | |
|---|---|
| panayensis | C2081X and cephamycin A and B |
| pluracidomyceticus | pluracidomycin A |
| rochei | cephamycin A and B |
| sioyaensis | MM 4550 and MM 13902 |
| sp. OA-6129 | OA-6129A |
| sp. KC-6643 | carpetimycin A |
| tokunomensis | asparenomycin A |
| viridochromogenes | cephamycin A and B |
| wadayamensis | WS-3442-D |

Many of the foregoing β-lactam antibiotic-producing organisms are used in the pharmaceutical industry for purposes of antibiotic production. The antibiotic-producing ability of these organisms can be increased and made more efficient by increasing the intracellular concentration of the antibiotic biosynthetic enzymes during the fermentation. The hydroxylase activity-encoding DNA compounds of the present invention can be used to construct expression vectors that, when transformed into the appropriate host cell, increase the intracellular concentration of hydroxylase activity of the transformed host cell and thereby increase the antibiotic-producing ability and efficiency of that cell.

A vector that will increase the intracellular concentration of hydroxylase activity of a given host cell into which the vector is transformed requires the following elements: 1) a hydroxylase activity-encoding DNA compound of the present invention; and 2) a promoter and translational activating sequence that not only function in the host cell to be transformed, but also are positioned in the correct orientation and position to drive expression of the hydroxylase activity-encoding DNA. Of course, stable transformants can only be obtained if the vector replicates, either as an extra-chromosomal element or integrated in the genomic DNA, in the host cell. Thus, a preferred vector might contain sequences that specifically direct replication or integration of the vector in the host cell. However, the presence of such specific replication or integration sequences is not absolutely required, because non-specific integration may occur when DNA is introduced into a host cell. A hydroxylase expression vector could also comprise an antibiotic resistance-conferring gene or some other element that provides a means of selecting for host cells which contain the vector, but such selectable elements may neither be necessary nor desired when the vector integrates into the chromosomal DNA of the host cell.

Many E. coli K12 strains contain an endogenous β-lactamase activity, probably encoded by the ampC locus. For this reason it is desirable to effect a partial purification of the hydroxylase polypeptide so that optimal hydroxylase activity is observed. Purification of the enzyme can be used to separate the endogenous E. coli β-lactamase activity from the desired hydroxylase activity. An alternative to the use of partial purification to overcome the deleterious effects of the β-lactamase is to use a strain defective in the production of this activity.

By providing the coding sequence of the hydroxylase gene of Streptomyces clavuligerus, the present invention provides hydroxylase expression vectors for any organism susceptible to transformation. The E. coli hydroxylase expression vectors described above illustrate the wide variety of expression vectors of the present invention. However, many of the preferred vectors of the invention are designed to drive expression of hydroxylase in a β-lactam antibiotic (including penicillins and cephalosporins) producing cell.

The vector described above and in Example 1 is merely illustrative of the wide variety of hydroxylase expression vectors provided by the present invention. U.S. Patent 4,885,251 describes the 5′ and 3′ regulatory signals of the Cephalosporium acremonium IPNS gene. The signals can be combined with the hydroxylase coding sequence of the present invention to yield hydroxylase expression vectors of the invention especially suited for use in Cephalosporium.

The hydroxylase expression vectors of the present invention are useful for increasing the intracellular concentration of hydroxylase activity in any cell, especially β-lactam antibiotic-producing cells. Plasmid pOW399 comprises the coding sequence of the hydroxylase gene of Streptomyces clavuligerus, so plasmid pOW399 can be used to construct vectors for increasing the copy number of the hydroxylase gene, which in turn increases the intracellular concentration of the enzyme. Because the hydroxylase coding sequence of the invention was isolated from a Streptomyces host cell, the hydroxylase coding sequence is particularly well-suited for use in expression vectors designed to drive high-level expression of hydroxylase activity in Streptomyces host cells. The literature is replete with techniques for constructing Streptomyces expression vectors and for transforming Streptomyces host cells. See, e.g., Garcia-Dominguez et al., 1987, Applied and Environmental Microbiology 53(6):1376-1381. The hydroxylase coding sequence of the invention can be readily incorporated

into an expression vector that comprises a Streptomyces promoter and replicon. A variety of known Streptomyces promoters and replicons are available for such use. Table II is an illustrative, but not comprehensive, listing of Streptomyces plasmids from which Streptomyces replicons can be obtained. Those skilled in the art recognize that, so long as the replicon function is not disrupted, all or part of the plasmids listed in the Table may be used to construct vectors that contain the hydroxylase gene of the present invention. The plasmid-containing host and depository accession number are also listed in Table II.

TABLE II

Streptomyces Plasmids

| Plasmid | Host | Accession Number |
|---|---|---|
| SCP2 | Streptomyces coelicolor A3(2) | NRRL 15042 |
| SCP2* | Streptomyces coelicolor M110 | NRRL 15041 |
| pEL7 | Streptomyces ambofaciens/pEL7 | NRRL 12523 |
| pUC6 | Streptomyces espinosus | NRRL 11439 |
| pUC3 | Streptomyces 3022A | NRRL 11441 |
| SLP1 | Streptomyces lividans | NCIB[1] 11417 |
| pNM100 | Streptomyces virginiae | NRRL 15156 |
| pEL103 | Streptomyces granuloruber A399 12.13/pEL103 | NRRL 12549 |
| pIJ702 | Streptomyces lividans | ATCC[2] 39155 |

[1] National Collection of Industrial Bacteria (NCIB), Torry Research Station, Post Office Box 31, 135 Abbey Road, Aberdeen AB98DG, Scotland, United Kingdom.

[2] American Type Culture Collection, Rockville, MD 20852.

The Streptomyces clavuligerus hydroxylase coding sequence of the invention can also be put under the control of transcription and translation activating sequences derived from other strains of Streptomyces, as well as from Penicillium, Cephalosporium, or any other host cell to construct a recombinant hydroxylase gene for use in the given organism.

The following Examples are provided to further illustrate and exemplify the present invention.

## Example 1

A lyophil of E. coli K12 JM109/pOW399 can be obtained from the Northern Regional Research Laboratories (NRRL), Peoria, IL 61604, under the accession number NRRL B-18658 (date of deposit: June 4, 1990) and used directly as the "culture" in the process described below.

### Production of S. clavuligerus Hydroxylase in E. coli K12 JM109/pOW399

### A. Expression of Hydroxylase in E. coli

E. coli K12 JM109/pOW399 was grown in 3 liters of TY broth and 5 μg/ml tetracycline at 30°C until the cells reached mid-log phase. The temperature of the mixture was raised to 42°C and incubation continued for about 4 more hours. The cl857 temperature-sensitive repressor of the lambda pL promoter, positioned to drive hydroxylase expression on plasmid pOW399, is inactivated at 42°C, thus allowing the expression of hydroxylase.

B. Isolation of Granules Containing Hydroxylase

Granules containing hydroxylase are isolated. First, the whole cells are centrifuged, then resuspended in water at 10°C. The cell slurry is homogenized in a Gaulin homogenizer at 8000 psig. The homogenized slurry is then diluted in water and agitated for 10 minutes, followed by adjustment of the pH to 8.4-8.6 with 10% sodium hydroxide. The mixture is then centrifuged. The solids represent the hydroxylase containing granules, which are frozen at -70°C until further use.

C. Preparation of Active Hydroxylase from Granules

Active hydroxylase was prepared from 0.5 g of washed granules by resuspending the granules in 8 ml folding buffer containing 0.5 M urea, 0.5 ml dithiothreitol (DTT) and 5 mM Tris base (tris(hydroxymethyl)aminomethane). The pH was not adjusted. When the granules were resuspended, the pH was raised to 11.7 with sodium hydroxide and the solution was stirred for 5 minutes. After stirring, the pH was lowered to 9.5 with hydrochloric acid. The mixture was then subjected to centrifugation at 48,000 x g for 10 minutes. The supernatant was saved for use in the hydroxylase assay.

The hydroxylase activity was determined by monitoring DAC formation from DAOC at 260 nm with HPLC as described by Dotzlaf, J.E., and Yeh, W.-K. (1987) J. Bacteriol., 169, 1611-1618. The assay mixture volume was 1 ml and contained 0.3 μmol of DAOC, 0.3 μmol of α-ketoglutarate, 0.1 μmol of ferrous sulfate, 0.25 μmol of ascorbate, 1 μmol of DTT, 0.05 μmol of adenosine triphosphate (ATP), and 8.4 μg of protein sample in 15 mM MOPS (3-[N-morpholino]propanesulfonic acid) buffer, pH 7.3. The enzymatic reaction was initiated by adding the DAOC and the reaction was conducted for 20 minutes at 29°C. DAC formation was linear with reaction time for up to 40 minutes. One unit of enzyme activity is defined as the amount of hydroxylase required to cause formation of one μmol of DAC per minute from DAOC under the above-described assay conditions.

The specific activity of the hydroxylase is defined as units per milligram of protein.

The protein content was determined by the method of Bradford using bovine serum albumin as the standard (Bradford, M.M., 1979, Anal. Biochem., 72, 248-254).

The hydroxylase produced from E. coli K12 JM109/pOW399 had a specific activity of 60-80 mUnits/mg protein.

## Claims

1. A DNA compound that comprises an isolated DNA sequence encoding hydroxylase activity of Streptomyces clavuligerus.

2. The DNA compound of Claim 1 which comprises an isolated DNA sequence encoding

H-MetAlaAspThrProValProIlePheAsnLeuAlaAlaLeuArgGluGlyAlaAspGln
GluLysPheArgGluCysValThrGlyMetGlyValPheTyrLeuThrGlyTyrGlyAla
GlyAspLysAspHisArgLeuAlaThrAspThrAlaMetAspPhePheAlaAsnGlyThr
GluAlaGluLysAlaAlaValThrThrAspValProThrMetArgArgGlyTyrSerAla
LeuGluAlaGluSerThrAlaGlnValThrArgThrGlySerTyrThrAspTyrSerMet
SerPheSerMetGlyIleSerGlyAsnValPheProSerProGluPheGluArgValTrp
ThrGluTyrPheAspLysLeuTyrAlaAlaAlaGlnGluThrAlaArgLeuValLeuThr
AlaSerGlyGlyTyrAspAlaGluIleValGlySerLeuAspGluLeuLeuAspAlaAsp
AlaSerGlyGlyTyrAspAlaGluIleValGlySerLeuAspGluLeuLeuAspAlaAsp
ProValLeuArgLeuArgTyrPheProGluValProGluHisArgSerAlaGluHisGlu
ProArgArgMetAlaProHisTyrAspLeuSerIleIleThrPheIleHisGlnThrPro
CysAlaAsnGlyPheValSerLeuGlnAlaGluIleGlyGlyGluLeuValSerLeuPro
ValValGluAspAlaValValValMetCysGlyAlaMetAlaProLeuAlaThrGlnGly
AlaLeuProAlaProArgHisHisValArgSerProGlyAlaGlyMetArgGluGlySer
AspArgThrSerSerValPhePheLeuArgProThrThrAspPheSerPheSerValAla
LysAlaArgSerTyrGlyLeuAlaValAspLeuAspMetGluThrAlaThrPheGlyAsp
TrpIleGlyThrAsnTyrValThrMetHisAlaLysAsnGluProGlnAlaGly-OH

wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue.

3. The DNA compound of Claim 2 which comprises the isolated DNA sequence:

```
1    ATGGCGGACA CGCCCGTACC GATCTTCAAC CTCGCCGCAC TGCGGGAAGG
51   CGCCGATCAG GAGAAGTTCC GCGAGTGCGT GACCGGGATG GGGGTCTTCT
101  ACCTCACCGG GTACGGCGCC GGGGATAAGG ACCACCGGCT GGCCACGGAC
151  ACGGCGATGG ACTTCTTCGC GAACGGCACC GAGGCCGAGA AGGCGGCCGT
201  GACCACGGAC GTCCCGACCA TGCGGCGCGG CTACTCCGCG CTGGAGGCCG
251  AGAGCACCGC CCAGGTGACC AGGACCGGTT CCTACACGGA CTACTCGATG
301  TCCTTCCCAT GGGCATCTCG GGCAACGTCT TCCCCTCGCC GGAGTTCGAG
351  CGGGTGTGGA CGGAGTACTT CGACAAGCTC TACGCGGCGG CCCAGGAGAC
401  GGCGCGGCTG GTGCTGACCG CGAGCGGCGG CTATGACGCG GAGATCGTCG
451  GAAGCCTGGA CGAGCTGCTG GACGCCGACC CCGTGCTGCG GCTGCGGTAC
501  TTCCCCGAGG TGCCCGAGCA CCGGTCCGCC GAGCACGAGC CGCGCCGGAT
551  GGCCCCGCAC TACGACCTGT CGATCATCAC CTTCATCCAC CAGACGCCGT
601  GCGCCAACGG CTTCGTCAGC CTCCAGGCCG AGATCGGCGG CGAACTGGTG
651  AGCCTGCCCG TCGTGGAGGA CGCCGTCGTC GTGATGTGCG GCGCGATGGC
701  CCCGCTGGCG ACCCAGGGCG CGCTGCCCGC GCCCCGGCAC CACGTCCGGT
751  CCCCCGGCGC CGGTATGCGC GAGGGCAGCG ACCGCACGTC GAGCGTCTTC
801  TTCCTGCGCC CCACGACCGA CTTCTCGTTC TCGGTGGCCA AGGCCCGCAG
851  CTACGGCCTC GCCGTCGACC TCGACATGGA GACGGCCACC TTCGGCGACT
901  GGATCGGCAC CAACTACGTC ACCATGCACG CGAAGAACGA GCCGCAGGCC
951  GGATGA
```

wherein A is a deoxyadenyl residue, G is a deoxyguanyl residue, C is a deoxycytidyl residue, and T is a thymidyl residue.

4. The ~2.5 kb EcoRI-BamHI restriction fragment of plasmid pOW399.

5. A recombinant DNA vector that comprises the isolated DNA sequence of Claim 1.

6. The recombinant DNA expression vector of Claim 5 that is plasmid pOW399.

7. A method for constructing a recombinant host cell capable of expressing hydroxylase activity of Streptomyces clavuligerus, said method comprising transforming a host cell with a recombinant DNA vector that comprises an isolated DNA sequence of Claim 1.

8. The method of Claim 7, wherein said recombinant host cell is selected from the group consisting of Escherichia, Cephalosporium, Streptomyces, Aspergillus, or Penicillium.

9. A method for expressing hydroxylase activity of Streptomyces clavuligerus in a recombinanthost cell, said method comprising culturing said transformed host cell of Claim 7 under conditions suitable for gene expression.

10. The method of Claim 9, wherein said recombinant host cell is selected from the group consisting of Escherichia, Cephalosporium, Streptomyces, Aspergillus, or Penicillium.

# Figure 1
# Beta-Lactam Biosynthesis

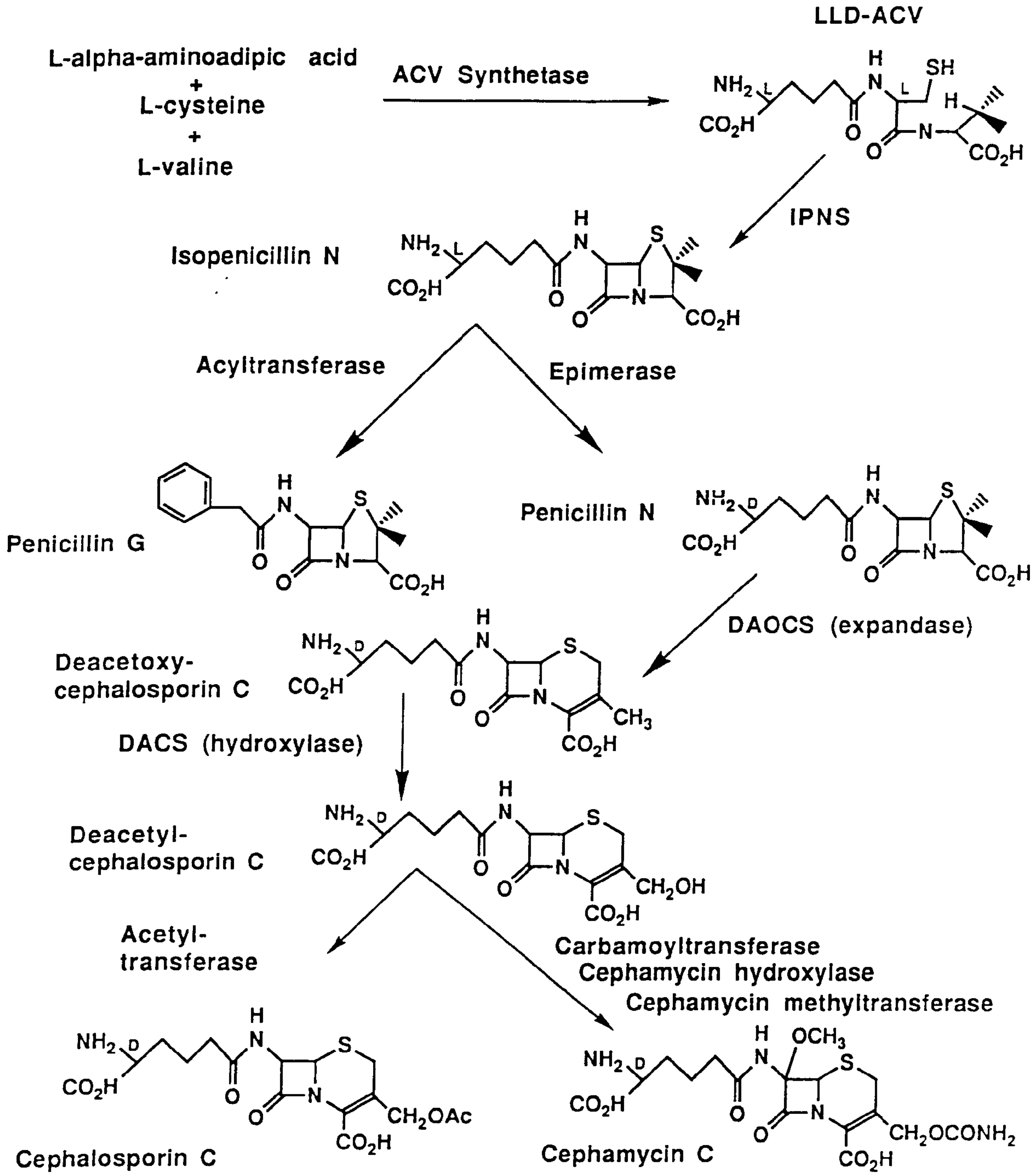


L-alpha-aminoadipic acid + L-cysteine + L-valine

LLD-ACV

Isopenicillin N

Penicillin G

Penicillin N

Deacetoxy-cephalosporin C

Deacetyl-cephalosporin C

Cephalosporin C

Cephamycin C

Figure 2